# EUROPÄISCHE PATENTANMELDUNG

(11) **EP 4 672 123 A1**
(43) Veröffentlichungstag der Anmeldung: **31.12.2025**
(21) Anmeldenummer: 24000079.4
(22) Anmeldetag: 24.06.2024
(51) Int. Cl.: G06Q 30/0251, A61B 5/00, G06Q 30/0601

(54) **VERFAHREN ZUR MESSUNG VON HAUTPARAMETERN FÜR DIE ANALYSE VON HAUTBEDÜRFNISSEN**

(71) Anmelder: Hallerbach, Bernd, 48324 Sendenhorst (DE)
(72) Erfinder: Die Erfindernennung liegt noch nicht vor

(57) **Zusammenfassung**

Ein Verfahren zur Messung von Hautparametern für die Analyse von Hautbedürfnissen mittels mindestens eines Messgerätes (19, 20), bei dem der Hauttyp eines Verbrauchers (16) von Kosmetika und/oder Arzneimitteln untersucht wird, wobei die Untersuchung des Hauttyps an einem von einem Anbieter (11) der Untersuchung und/oder von dem Verbraucher (23) der Kosmetika und/oder Arzneimittel frei bestimmbaren Standort (10) durchgeführt wird, wobei das Messgerät (19, 20) zum frei bestimmbaren Standort (10) mittels eines Transportmittels (12) in Gestalt eines Koffers oder einer Transportbox überführt wird und wobei das Ergebnis der Hautuntersuchung visuell dargestellt wird, wobei in das Transportmittel (12) eine Computereinrichtung (14) integriert wird und die Computereinrichtung (14) mit dem Messgerät (19, 20) verbunden wird, wobei mittels einer Software die Ergebnisse der Hautuntersuchung auf der Bildfläche (16) eines Monitors (15) dargestellt werden und mehrere Messparameter während der Untersuchung verwendet werden. Um das Verfahren derart weiterzuentwickeln, dass aufgrund der umfangreichen Hautanalyse dem Verbraucher auch schnellstmöglich eine differenzierte Produktpalette zur Verfügung steht, die auf den Bedarf des Verbrauchers als Folge der Hautuntersuchung bzw. Hautanalyse abgestimmt sind, wird vorgeschlagen, dass eine Kommunikation über eine erste Schnittstelle (21) zwischen der Computereinrichtung (14) und einer App (24), die auf einem Endgerät (22) des Verbrauchers (23) installiert und ausgeführt wird, stattfindet, wobei die App (24) programmtechnisch derart eingerichtet ist, dass auch eine Kommunikation über eine zweite Schnittstelle (25) zwischen der App (24) und einer Computereinrichtung (26) ausschließlich eines Handelsunternehmens (28) für Kosmetika und/oder Arzneimittel stattfindet.

## Beschreibung

Die Erfindung betrifft ein Verfahren zur Messung von Hautparametern für die Analyse von Hautbedürfnissen gemäß dem Oberbegriff des Anspruchs 1.

Ein Verfahren der eingangs genannten Art ist aus der EP 3 103 361 B1 bekannt.

Bei diesem bekannten Verfahren wird die Untersuchung des Hauttyps an einem von einem Anbieter der Untersuchung und/oder von dem Verbraucher von Kosmetika frei bestimmbaren Standort durchgeführt wird, wobei die hierzu erforderlichen Messgeräte zum frei bestimmbaren Standort mittels eines Transportmittels überführt werden. Hierbei werden die Messgeräte in einem Koffer als Transportmittel überführt. In der EP 3 103 361 B1 ist weiterhin vorgesehen, dass in das Transportmittel eine Computereinrichtung integriert ist. Die Computereinrichtung kann dabei mit den Messgeräten verbunden werden. Hierdurch können bei Vorhandensein einer entsprechenden Software die Ergebnisse der Hautuntersuchungen auch auf der Bildfläche eines Monitors dargestellt werden. Ein erster Vorteil des in der EP 3 103 361 B1 beschriebenen Verfahrens ist es also, die Untersuchung des Hauttyps des Verbrauchers mobil durchführen zu lassen, um den Verbraucher besser zu erreichen. Durch das bekannte Verfahren werden also mobile Verkaufspunkte geschaffen, an denen vorab der Hauttyp eines Verbrauchers von Kosmetika untersucht wird.

Ein weiterer Vorteil des in der EP 3 103 361 B1 offenbarten Verfahrens ist es, dass mehrere Messparameter während der Untersuchung verwendet werden können. Hierdurch ist eine umfangreiche mobile Analyse des Hauttyps und somit auch Beratung des Verbrauchers gewährleistet. Hierzu ist in der EP 3 103 361 B1 die in das Transportmittel integrierte Computereinrichtung vorgesehen, wobei die mehrere Messparameter umfassenden Ergebnisse der Hautuntersuchung mittels einer Software auf der Bildfläche eines Monitors dargestellt werden.

Somit führt das aus der EP 3 103 361 B1 bekannte Verfahren nicht nur dazu, mehrere Messparameter mithilfe mehrerer Messgeräte für die Untersuchung zu verwenden, sondern auch dazu, mithilfe einer integrierten Computereinrichtung und einer Software das mehrere Parameter umfassende Ergebnis der Hautuntersuchung auf einem Monitor einer Computereinrichtung an einem beliebigen Ort darzustellen.

Die umfangreichere Analyse der Haut, die durch das in EP 3 103 361 B1 offenbarte Verfahren gegeben ist, kann auch zu einer differenzierteren Produktempfehlung führen. So kann beispielsweise aufgrund der umfangreichen Hautanalyse sowohl eine Produktempfehlung zur Verbesserung der Feuchtigkeit der Haut als auch zur Reduzierung des Hautfetts gegeben werden.

Die entsprechenden Produkte sollten dem Verbraucher selbstverständlich schnellstmöglich zur Verfügung stehen, auch wenn er mobil unterwegs ist.

Es ist deshalb Aufgabe der vorliegenden Erfindung, das Verfahren der eingangs genannten Art derart weiterzuentwickeln, dass aufgrund der umfangreichen Hautanalyse dem Verbraucher auch schnellstmöglich eine differenzierte Produktpalette zur Verfügung steht, die auf den Bedarf des Verbrauchers als Folge der Hautuntersuchung bzw. Hautanalyse abgestimmt sind.

Diese Aufgabe wird mit den Merkmalen des Anspruchs 1 gelöst. Vorteilhafte Weiterbildungen der Erfindung ergeben sich aus den abhängigen Ansprüchen.

Die Erfindung sieht vor, dass eine Kommunikation über eine erste Schnittstelle zwischen der Computereinrichtung und einer App, die auf einem Endgerät des Verbrauchers installiert und ausgeführt wird, stattfindet, wobei die App programmtechnisch derart eingerichtet ist, dass auch eine Kommunikation über eine zweite Schnittstelle zwischen der App und einer Computereinrichtung ausschließlich eines Handelsunternehmens für Kosmetika und/oder Arzneimittel stattfindet.

Gemäß der Erfindung wird also auch nur mit einem einzigen Handelsunternehmen für Kosmetika und Arzneimittel kommuniziert und zwar direkt. Die Erfindung geht infolgedessen auch über das Verfahren hinaus, das beispielsweise in der EP 3 595 508 B1 beschrieben wird. Bei dem in der der EP 3 595 508 B1 offenbarten Verfahren ist von vornherein nur die Ermittlung des Hautfeuchtigkeitsgehalts und somit die Verwendung eines Messparameters und dessen visuelle Darstellung vorgesehen. Eine direkte Kommunikation mit einem Handelsunternehmen für Kosmetika und Arzneimittel findet in der EP 3 595 508 B1 nicht statt. Vielmehr werden in der EP 3 595 508 B1 über eine App entsprechende Handelsunternehmen für Kosmetika und Arzneimittel erst wegen einer vorhandenen Produktempfehlung angefragt, d.h. Verbindungen zu verschiedenen Handelsunternehmen sind möglich. Auf diese Weise ist jedoch nicht gewährleistet, dass eine differenzierte Produktpalette schnellstmöglich zur Verfügung steht.

Gemäß der Erfindung ist die App programmtechnisch jedoch derart eingerichtet, dass auch eine Kommunikation über eine zweite Schnittstelle zwischen der App und einer Computereinrichtung ausschließlich eines Handelsunternehmens für Kosmetika und Arzneimittel stattfindet. Somit wird die Voraussetzung geschaffen, dass von vornherein Handelsunternehmen zur Verfügung stehen oder eingerichtet werden können, die das gewünschte Produktportfolio, die aus den umfangreichen Hautanalysen resultieren, anbieten und vertreiben können.

Die Erfindung bietet also den besonderen Vorteil, dass die Dienstleistung der mobilen umfangreichen Hautanalyse weiter optimiert wird, indem eine differenzierte Produktpalette eines Handelsunternehmens, die auf die Ergebnisse der Hautanalyse und die damit einhergehenden Produktempfehlungen abgestimmt ist, prinzipiell schnell zur Verfügung gestellt werden kann. Der Anbieter der mobilen Hautanalyse kann dabei in Kooperation mit dem Handelsunternehmen stehen.

Die App kann vorzugsweise verschiedene Arten von Schnittstellen nutzen, wobei die Schnittstellen mit der App impliziert, dass auch die Computereinrichtungen des Anbieters der mobilen Hautanalyse bzw. der Transportmittels und des Handelsunternehmens mit ihren Computerprogrammen zur Hautanalyse und E-Commerce programmtechnisch derart eingerichtet sind, dass über die Schnittstellen mit der App kommuniziert werden kann.

Für die Schnittstelle zwischen der Computereinrichtung des Transportmittels und der App kann vorteilhafterweise eine Netzwerkschnittstelle verwendet werden. Die App kann somit über das Internet kommunizieren, um die Verbindung zur Computereinrichtung herzustellen und Daten zu senden und zu empfangen. Diese Schnittstelle kann auf verschiedenen Protokollen basieren, z.B. HTTP oder Bluetooth. Über diese Schnittstelle können vorteilhafterweise Dienstleistungen angeboten und in Anspruch genommen worden. Beispielsweise können über die App, die entsprechend programmtechnisch eingerichtet ist, Buchungen von Terminen für die Hautanalyse und Ergebnisse der Hautanalyse gespeichert und abgerufen werden.

Vorzugsweise kann für die Schnittstelle zwischen der App und dem Handelsunternehmen eine E-Commerce-Schnittstelle benutzt werden. Die App ist dabei programmtechnisch so eingerichtet, dass sie E-Commerce-Funktionalitäten bereitstellt, d.h. mit einem Handelsunternehmen kommunizieren und Funktionen wie das Hinzufügen von Produkten zum Warenkorb oder das Abschließen von Bestellungen ermöglichen kann. E-Commerce-Schnittstellen sind aus dem Stand der Technik hinreichend bekannt.

Das Messgerät kann mit einer externen Computereinrichtung verbunden werden. Der Anbieter der Untersuchungen kann so erst an dem gewählten Standort die Verbindung zwischen Messgerät und Computereinrichtung herstellen, was insbesondere bei größeren Computereinrichtungen geboten ist.

Um die Ergebnisse auch in Papierform vorliegen zu haben, ist es zweckmäßig, dass die Computereinrichtung mit einer Druckereinrichtung verbunden wird.

Mehrere Messgeräte können zwecks eine umfangreichen Hautanalyse bei der Untersuchung eingesetzt werden. Beispielsweise können Messgeräte für die Messung der Feuchtigkeit der Haut, von FettISebum, der Pigmentierung und der Hautrötung etc. zum Einsatz kommen.

Eine weitere vorteilhafte Weiterbildung der Erfindung sieht vor, dass ein zusätzliches Transportmittel eingesetzt wird, wobei das zusätzliche Transportmittel motorisch angetrieben wird. Durch diese erfindungsgemäße Maßnahme kann ein Messgerät, das beispielsweise in einem Koffer transportiert wird, zusätzlich beispielsweise mittels eines Kraftfahrzeugs befördert werden. Hierdurch wird neben der schonenden Beförderung des Messgeräts, beispielsweise in einer Transportbox, auch die schnelle Beförderung, beispielsweise mittels eines Kraftfahrzeugs, gewährleistet.

Eine vorteilhafte Weiterbildung der Erfindung sieht vor, dass die App programmtechnisch derart eingerichtet ist, dass sie über eine zweite Schnittstelle mit einer weiteren App des Endgeräts kommuniziert. Hierbei handelt es sich vorteilhafterweise um eine Programmierschnittstelle, die auch im Stand der Technik unter der Bezeichnung API bekannt ist, d.h. eine Schnittstelle ist, die von der App, welche die Schnittstelle mit der Computereinrichtung des Transportmittels aufweist, bereitgestellt wird. Hierdurch eröffnet die App einer weiteren App des Endgeräts des Verbrauchers die Möglichkeit, der weiteren App Zugriff auf ihre Funktionalitäten zu ermöglichen. Beispielsweise könnte die App eine API bereitstellen, damit die weitere App die Produktempfehlungen zur weiteren Verwendung, beispielsweise für eine Bestellung bei dem Handelsunternehmen für Kosmetika und Arzneimittel, erhält. Diese kann sinnvoll sein, wenn das Handelsunternehmen unter einem weiteren Namen firmiert und alternative Schnittstelle zur Verfügung stellt.

Eine praktikable Variante der Erfindung sieht vor, dass die App programmtechnisch derart eingerichtet ist, dass sie über eine Schnittstelle mit einer Datenbank kommuniziert. Eine derartige Schnittstelle kann sinnvoll sein, wenn der Verbraucher weitere Informationen über das empfohlene Produkt erhalten möchte, die in entsprechenden Datenbanken enthalten sind. Auch Datenbankschnittstellen sind im Stand der Technik hinreichend bekannt. Diese Schnittstellen können in verschiedenen Formen vorliegen, beispielsweise als SQL-Schnittstelle für relationale Datenbanken oder als NoSOL-Schnittstelle für nicht relationale Datenbanken.

Weitere Ziele, Vorteile, Merkmale und Anwendungsmöglichkeiten der vorliegenden Erfindung ergeben sich aus der nachfolgenden Beschreibung eines Ausführungsbeispiels anhand der Zeichnung.

Es zeigen in schematischer Darstellung:

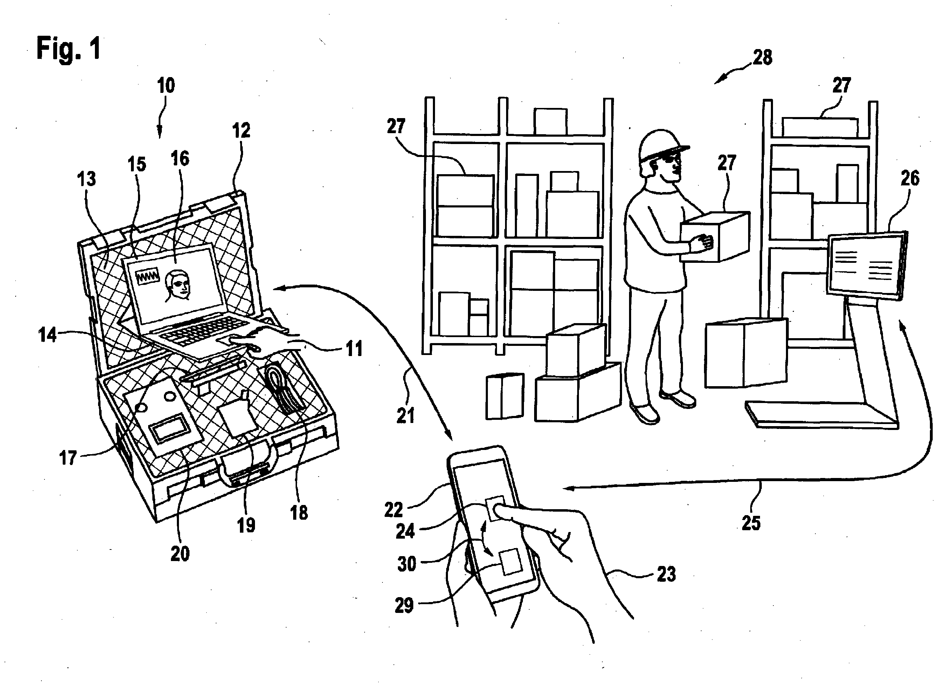

- Fig. 1: ein Verfahren gemäß der Erfindung.

Figur 1 zeigt das erfindungsgemäße Verfahren, das mit der Messung von Hautparametern für die Analyse von Hautbedürfnissen beginnt. Hierzu wird zunächst mittels der in Figur 1 gezeigten und in dem Transportmittel 12, hier ein Transportkoffer, befindlichen Messgeräte 19, 20 von einem Anbieter 11 wie einem Apotheker oder Arzt eine Messung und Analyse des Hauttyps eines Verbrauchers 23 vorgenommen. Der Verbraucher 23 verfügt über ein Endgerät 22 in Gestalt eines Smartphones.

Die Untersuchung des Hauttyps wird an einem von dem Anbieter 11 oder Verbraucher 23 frei bestimmbaren Standort 10 durchgeführt.

Um die Untersuchung des Hauttyps an dem frei bestimmbaren Ort 10 durchzuführen, werden die in Figur 1 gezeigten Messgeräte 19, 20 in dem Transportmittel 12 zum frei bestimmbaren Standort 10 mittels des Transportmittels 12 überführt. Zu dem Messgerät 20 gehört der Messstift 17, der mit dem Messgerät 20 verbunden werden kann. Mit dem Messgerät 20 wird die Feuchtigkeit der Haut des Verbrauchers 23 ermittelt. Hierzu wird die Spitze des Stifts 17 in einem unmittelbaren Kontakt mit der Haut des Verbrauchers 23 gebracht.

Wie aus Figur 1 weiter hervorgeht, weist das Transportmittel 10 neben einem Ersatzkabel 17 das Messgerät 19 auf, das sich für die Messung des Hautfetts des Verbrauchers 23 eignet. Die Messung des Hautfetts erfolgt über eine photometrische Messung. Die photometrische Messung des Hautfetts ergänzt die Feuchtigkeitsmessung, um den individuelle Basishauttyp des Verbrauchers 23 zu ermitteln. Insgesamt werden somit mehrere Messparameter für die Untersuchung der Haut des Verbrauchers 23 verwendet.

Um das Ergebnis der Hautuntersuchung visuell darzustellen, ist in eine Wand 13 des Transportmittels 12 eine Computereinrichtung 14 einklappbar integriert. Während der der Untersuchung der Haut des Verbrauchers 23 und der Messung der Hautparameter wird die Computereinrichtung 14 mit den Messgeräten 19, 20 verbunden. Mittels einer Software werden die Ergebnisse der Hautuntersuchung auf der Bildfläche 16 des Monitors 15 der Computereinrichtung 14 dargestellt, wodurch auch mehrere Messparameter auf der Bildfläche 16 des Monitors 15 visualisiert werden.

Aus der Hautuntersuchung geht eine Produktempfehlung des Anbieters 11 hervor, d.h. dem Verbraucher 23 werden gewisse Kosmetika bzw. Arzneimittel empfohlen, um den Hautzustand des Verbrauchers 23 zu verbessern. Diese Produktempfehlung kann auch eine ärztliche Verordnung sein.

Um dem Verbraucher 23 diese Produktempfehlung mitzuteilen, findet eine Kommunikation über eine erste Schnittstelle 21 zwischen der Computereinrichtung 14 und der App 24, die auf dem Endgerät 22 des Verbrauchers 23 installiert und ausgeführt wird, statt. Die App 24 und die Computereinrichtung 14 sind dabei programmtechnisch derart eingerichtet, dass eine solche Kommunikation über die Schnittstelle 21 auch stattfinden kann.

Der Verbraucher 23 hat nunmehr die Möglichkeit die empfohlenen Kosmetika bzw. Arzneimittel aus der breiten Produktpallette, die speziell auf die Ergebnisse der Hautuntersuchung abgestimmt sind, bei einem hierfür spezialisierten Handelsunternehmen 28 zu bestellen.

Hierzu ist die App 24 programmtechnisch derart eingerichtet ist, dass auch eine Kommunikation über eine zweite Schnittstelle 25 zwischen der App 24 und einer Computereinrichtung 26 ausschließlich eines Handelsunternehmens 28 für Kosmetika und Arzneimittel stattfindet. Für die Bestellung wird also auch nur mit dem Handelsunternehmen 28 für Kosmetika und Arzneimittel kommuniziert und zwar direkt.

Nach Eingang der Bestellung des Verbrauchers 23 findet in dem Handelsunternehmen die Abfertigung statt, d.h. die bestellten Kosmetika bzw. die Arzneimittel werden verpackt und die Pakete 27 werden zum Versand fertig gemacht und zum Verbraucher 23 transportiert.

Wie aus Figur 1 weiter hervorgeht, ist die App 24 weiterhin programmtechnisch derart eingerichtet, dass sie über eine zweite Schnittstelle 30 mit der weiteren App 29 des Endgeräts 22 kommuniziert. Hierbei handelt es sich um eine Programmierschnittstelle, die auch unter der Bezeichnung API bekannt ist, d.h. eine Schnittstelle ist, die von der App 24 bereitgestellt wird. Hierdurch eröffnet die App 24 der weiteren App 29 des Endgeräts 22 des Verbrauchers 23 die Möglichkeit, der weiteren App 29 Zugriff auf ihre Funktionalitäten zu ermöglichen.

## Patentansprüche

1. Verfahren zur Messung von Hautparametern für die Analyse von Hautbedürfnissen mittels mindestens eines Messgerätes (19, 20), bei dem der Hauttyp eines Verbrauchers (23) von Kosmetika und/oder Arzneimitteln untersucht wird, wobei die Untersuchung des Hauttyps an einem von einem Anbieter (11) der Untersuchung und/oder von dem Verbraucher (23) der Kosmetika undloder Arzneimittel frei bestimmbaren Standort (10) durchgeführt wird, wobei das Messgerät (19, 20) zum frei bestimmbaren Standort (10) mittels eines Transportmittels (12) in Gestalt eines Koffers oder einer Transportbox überführt wird und wobei das Ergebnis der Hautuntersuchung visuell dargestellt wird, wobei in das Transportmittel (12) eine Computereinrichtung (14) integriert wird und die Computereinrichtung (14) mit dem Messgerät (19, 20) verbunden wird, wobei mittels einer Software die Ergebnisse der Hautuntersuchung auf der Bildfläche (16) eines Monitors (15) dargestellt werden und mehrere Messparameter während der Untersuchung verwendet werden, **dadurch gekennzeichnet, dass** eine Kommunikation über eine erste Schnittstelle (21) zwischen der Computereinrichtung (14) und einer App (24), die auf einem Endgerät (22) des Verbrauchers (23) installiert und ausgeführt wird, stattfindet, wobei die App (24) programmtechnisch derart eingerichtet ist, dass auch eine Kommunikation über eine zweite Schnittstelle (25) zwischen der App (24) und einer Computereinrichtung (26) ausschließlich eines Handelsunternehmens (28) für Kosmetika undloder Arzneimittel stattfindet.

2. Verfahren nach Anspruch 1, **dadurch gekennzeichnet, dass** die Computereinrichtung (14) mit einer Druckereinrichtung verbunden wird.

3. Verfahren nach einem der vorhergehenden Ansprüche, **dadurch gekennzeichnet, dass** mehrere Messgeräte (19, 20) bei der Untersuchung eingesetzt werden.

4. Verfahren nach einem der vorhergehenden Ansprüche, **dadurch gekennzeichnet, dass** ein zusätzliches Transportmittel eingesetzt wird, wobei das zusätzliche Transportmittel motorisch angetrieben wird.

5. Verfahren nach einem der vorhergehenden Ansprüche **dadurch gekennzeichnet, dass** die App (24) programmtechnisch derart eingerichtet ist, dass sie über ein zweite Schnittstelle (25) mit einer weiteren App (29) des Endgeräts (22) kommuniziert.

6. Verfahren nach einem der vorhergehenden Ansprüche, **dadurch gekennzeichnet, dass** die App (24) programmtechnisch derart eingerichtet ist, dass sie über eine Schnittstelle mit einer Datenbank kommuniziert.
